# EUROPEAN PATENT SPECIFICATION

(11) **EP 1 326 623 B1**
(45) Date of publication and mention of the grant of the patent: **25.04.2007**
(21) Application number: 01981111.6
(22) Date of filing: 19.10.2001
(51) Int. Cl.: A61K 36/78, A61K 31/40, A61P 25/28

(54) **SAURURUS CHINENSIS EXTRACT FOR PREVENTION AND TREATMENT OF NEURODEGENERATIVE DISEASE**
SAURURUS CHINENSIS-EXTRAKT ZUR PROPHYLAXIS UND BEHANDLUNG NEURODEGENERATIVER KRANKHEITEN
EXTRAIT DE SAURURUS CHINENSIS UTILISE DANS LA PREVENTION ET LE TRAITEMENT DE MALADIES NEURODEGENERATIVES

(30) Priority: 19.10.2000 KR 2000061687; 19.10.2000 KR 2000061688
(43) Date of publication of application: 16.07.2003
(73) Proprietor: Elcom Bio Technology, Co., LTD., Seoul 143-802 (KR)
(72) Inventor: KIM, Young-Joong, Gwangjin-gu, Seoul 143-752 (KR); SUNG, Sang-Hyun, Seoul 151-013 (KR); KIM, So-Ra, Gwanak-gu, Seoul 151-057 (KR)
(74) Representative: Ritthaler, Wolfgang
(86) International application number: PCT/KR2001/001766
(87) International publication number: WO 2002/032443

(56) References cited:
- JP-A- 10 251 155
- US-A- 4 619 943
- US-A- 4 782 077
- YAKHAK HOECHI vol. 51, no. 6, 1987, SAMJIN PHARM. CO., LTD., pages 704 - 708

## Description

### Technical field

The invention relates to the use of a compound of the following formula (I) in the manufacture of medicines for prevention and treatment of stroke or Alzheimer's disease 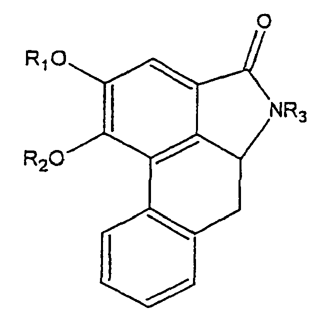 wherein R₁ is H, C₁₋₄ alkyl or the group of 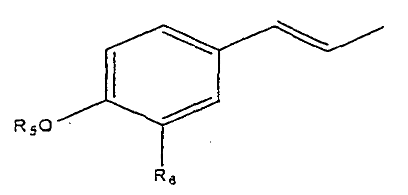 (in which R₅ is H or C₁₋₄ alkyl, R₆ is H, OH or C₁₋₄ alkyl),
R₂ is C₁₋₄ alkyl and
R₃ is C₁₋₄ alkyl.

### Background of the technology

Recently, patients suffering from neurodegenerative disorders are rapidly increasing in parallel with the increase of aged persons. Typical neurodegenerative disorder is Alzheimer's disease. The main symptoms of the Alheimer's disease are loss of memory and decrease of learning power. Families suffer in addition to the patients. Therefore, it becomes social problem. Interest in prevention and treatment of Alzheimer's disease is daily increasing. In addition, although according to rapid developement of biotechnology and science & technology, quility of living of human being became increased, outbreak of disease of aged persons is also increasing. The outbreak of neurodegenerative disorders such as stroke by the 2nd symptoms is also increasing. Though such outbreak of neurodegenerative disorders is increasing based on various origins, no drug which can be effectively prevent and treat such disorders was developed until now. Neurodegenerative disorders can be occurred by neurodegeneration caused by concussion of the brain and aging, 2nd phenomena like circulatory disorders and neurodegenerative disorders caused by various physical or mechanical factors like traffic accident, workman's accident, CO-poisoning.(Rothman, S. M. (1984) Synaptic release of excitatory amino acid neurotransmitter mediates anoxic neuronal death. J. Neurosci. 4: 1884-1891, and Weiloch, T. (1985) Hypoglycemia-induced neuronal damage prevented by an NMDA antagonists. Science 230: 681-683).

By above reasons, in case blood flow into brain is decreased or interrupted, brain tissue becomes hypoxic or low-glucosic. Then the brain can not perform normal metabolism and becomes irrevocably injured. Such cases can be often seen in stroke, trauma, ischemic injury and neurodegenerative disorders. (Benveniste, H., Drejer, J., Schousboe, A. and Diemer, N. H. (1984) Elevation of the extracellular concentrations of glutamate and aspartate in rat hippocampus during transient cerebral ischemia monitored by intracerebral microdialysis. J. Neurochem. 43: 1369-1374 and Hagberg , H., Lehmann, A., Saucberg, M., Nystrom, B., Jacobson, I. and Hamberger, A. (1985) Ischemia-induced shift of inhibitory and excitatory amino acids from intra- to extracellular compartments. J. Cereb. Blood Flow & Metab. 5: 413-419).

Injury of brain tissue can largely be divided into 2 kinds of mechanism. The one is originated from increase of free excitatory amino acid based on voltage change of cell membrane. The other is originated from direct oxidative stress. (Choi, D. W. (1988) Glutamate neurotoxicity and disease of the nervous system. Neuron 1: 623-634 and Coyle, J. T. and Purrfarcken, P. (1993) Oxidative stress, glutamate and neurodegenerative disorders. Science 262: 689-695). According to recent study, glutamate is regarded as original material which occurs oxidative stress by activating inotrophic receiptor. Therefore, glutamate is regarded as origin of outbreak of stroke, trauma, ischemic injury. (Halliwell, B and Gutteridge J. M. (1985a) Oxygen radicals and the nervous system. Trends Neurosci. 5: 22-26 and Halliwell, B and Gutteridge J. M. (1985b) The importance of free radicals and catalytic metal ions in human diseases. Mol. Aspects Med. 8: 89-193). In case brain blood flow becomes decreased, glutamate liberation at synapsis becomes increased and the flow of glutamate into neuron becomes decreased. Accordingly, the concentration of glutamate becomes rapidly increased outside cell. At last, neurons become dead. (Benveniste, H., Drejer, J., Schousboe, A. and Diemer, N. H. (1984) Elevation of the extracellular concentrations of glutamate and aspartate in rat hippocampus during transient cerebral ischemia monitored by intracerebral microdialysis. J. Neurochem. 43: 1369-1374 and Hagberg, H., Lehmann, A., Saucberg, M., Nystrom, B., Jacobson, I. and Hamberger, A. (1985) Ischemia-induced shift of inhibitory and excitatory amino acids from intra- to extracellular compartments. J. Cereb. Blood Flow & Metab. 5: 413-419). Such neurotoxicity based on glutamate is divided into "acute toxicity" in which toxicity is strongly and rapidly revealed and "chronic toxicity" in which neurons are exposed at low concentration of glutamate or exposed at high concentration of glutamate for a short time. (Choi, D. W. (1988) Glutamate neurotoxicity and disease of the nervous system. Neuron 1: 623-634). Acute toxicity begins when Ca²⁺ which exists outside cells is overflowed into cells and in order to maitaining osmotic pressure, ions such as Na⁺, K⁺ and Cl⁻ are flowed into cells and cells become to burst out to death. (Kauppisen, R. A., McMahonm, H. T. and Nicholls, D. G. (1988) Ca²⁺-dependent and Ca²⁺-independent glutamate release, energy status and cytosolic free Ca²⁺ concentration in isolated nerve terminals following metabolic inhibition: Possible relevance to hypoglycemia and anoxia, Neurosci. 27: 175-182 and Garthwaite, G. and Garthwaite, J. (1988) In vitro neurotoxicity of excitatory acid] analogues during cerebellar development, J. Neurosci. 17: 755-767).

Chronic toxicity begins when N-methyl-D-aspartate(NMDA), the glutamate receipt, and non-NMDA become activated and Ca²⁺ is flowed into cells and by this Ca²⁺-dependent enzymes are activated and at last, cells become dead. (Coyle, J. T. and Purrfarcken, P. (1993) Oxidative stress, glutamate and neurodegenerative disorders. Science 262: 689-695.). That is, as NMDA and non-NMDA are activated by glutamate, Ca²⁺ is flowed into cells and phopholipase A2, the Ca²⁺-dependent enzyme is abnormally activated, the formation of multi-free fatty acid is increased and therefore, free radicals such as active oxygen and/or active hydrogen peroxide are abnormally increased and accelerate peroxidation of cells and at last, cells die. (Coyle, J. T. and Purrfarcken, P. (1993) Oxidative stress, glutamate and neurodegenerative disorders. Science 262: 689-695 Halliwell, B and Gutteridge J. M. (1985a) Oxygen radicals and the nervous system. Trends Neurosci. 5: 22-26 Halliwell, B and Gutteridge J. M. (1985b) The importance of free radicals and catalytic metal ions in human diseases. Mol. Aspects Med. 8: 89-193 Siesjo, B. K. and Wieloch, J. (1985) Cerebral metabolism in ischaemia: neurochemical basis for therapy. Br. J. Anaesth. 47: 47-62 Sladeczek, F., Pin, J. P., Recasens, M., Bokaerk, J. and Weiss, S. (1985) Glutamate stimulates inositol phosphate formation in striatal neurones. Nature 317: 717-719 and Choi, D. W. and Koh, J. (1987) Ionic dependence of glutamate neurotoxicity. J. Neurosci. 7: 369-379). When NMDA receptor is activated, the activation of nitric oxide-synthesizing enzyme is increased and nitric oxide is over-formed. Thus formed nitric oxide accelerates peroxidation of cells, too. (Strijbos, P. J. L. M. Leach, M. J. and Garthwaite, J. (1996) Vicious cycle involving Na+ channels, glutamate release and NMDA receptors mediates delayed neurodegeneration through nitric oxide formation. J. Neurosci. 16: 5004-5013). Neurotoxicity can be prevented by antagonist of glutamate, sodium- or calcium-channel blocker, antioxidant, free radical blocker and anti-protein enzyme which suppresses the conversion from xanthin dehydrogenase to xanthin oxidase. Especially antagonist against NMDA receptor is effective.(Choi, D. W., Koh, J. and Peters, S. (1988) Pharmacology of glutamate neurotoxicity in cortical cell culture: attenuation by NMDA antagonists. J. Neurosci. 8: 185-196 and Olney, J. W. and Sharpe, I. G. (1969) Brain lesions in an infant rhesus monkey treated with monosodium glutamate. Science 166: 368-388).

In order to develope drug for Alzheimer's disease, the important disease which brings athymia, the directions of study are oriented to the both. That is, in the case of glutamate neurons, function is accentuated(use of effective drug) or the function is suppressed(use of antagonist). That is, when the content of glutamate is decreased by injury of glutamate neurons, memory and learning power is decreased. In the case of patients of Alzheimer's disease, the number of NMDA receptor, the glutamate receiptor is decreased to 75-87% of NMDA receiptor and to 45-69% of non-N-NMA receiptor. (Cowburn, R., Hardy, J., Roberts, P. and Briggs, R. (1988) Regional distribution of pre- and postsynaptic glutamatergic function in Alzheimer's disease. Brain Res. 452: 403-407 Greenamyre, J. T., Penney, J. B., D'Amato, C. J. and Young A. B. (1987) Dementia of the Alzheimer's type: Changes in hippocampal L-[3H] glutamate binding, J. Neurochem. 48: 543-551 and Chalmers, D. T., Dewar, D., Graham, D. L, Brooks, D. N. and McCulloch, J. (1990) Differential alternations of cortical glutamatergic binding sites in senile dementia of the Alzheimer type. Proc. Natl. Acad. Sci. USA 87: 1352-1356). And glutamate receptor is concentrated in brain area which is known to concern with memory and learning power. So, loss of memory and decrease of learning power are regarded to be effected by the abnormality of glutamate receptor. (Izquierdo, L (1991) Role of NMDA receptors in memory. Trends Pharm. Sci. 12: 260- 265). At the time of memorizing and learning, when calcium ion is increased and protein-lysing enzyme is activated, glutamate receptor is increased. As a result, structural and chemical changes in synapsis arise and human being can memorize and learn. This is the most possible hypothesis. (Thompson, R. F. (1986) The neurobiology of learning and memory. Science 233: 941- 947). The main symptoms of Alzheimer's disease is loss of memory. It is known that efficient drug for glutamate has an important role in memory and learning power. It is studied that by administrating the efficient drug for glutamate, efficient drug can be used for the treatment of loss of memory. Some efficient drugs have efficacy. (Willetts, J., Balster, R. L., Leander, J. D. (1990) The behavioral pharmacology of NMDA receptor antagonists. Trends Pharmacol Sci 11: 423-428). However, in a patient of Alzheimer's disease, re-adsorption of glutamate at pre-end of synapsis of nerve of cortex of cerebrum and hippocampus can not be done harmoniously. Content of decarboxylase which metabolizes glutamate and synthesizes GABA is decreased and as a result, content of glutamate in synapsis becomes increased. (Willetts, J., Balster, R. L., Leander, J. D. (1990) The behavioral pharmacology of NMDA receptor antagonists. Trends Pharmacol Sci 11: 423-428).

The present inventors referenced influences of chinese drugs or raw drugs to be known to have effects against neurodegenerative disdorders like stroke or failure of memory by using primary cultured rat cortical cells, based on the above literature and considerations. Total methanol extract was administered to primary cultured rat cortical cells and large amount of glutamate is administered and tried to know effects of the extract to neuron death. As the results, we found that total methanol extract of Saururus chinensis had significant effect on neuroprotective activity. In addition, alkaloids of aristolactam derivatives isolated from Saururus chinensis has significant neuroprotective activity.

Saururus chinensis is a perennial vegetable which belongs to Saururaceae. The whole plant, root or leaf has been used among civilians as treatment drug against diuresis, edema, gonorrhea, hepatitis, pneumonia and hypertension.

Study on ingredients of Saururus chinensis was mainly carried out on flavonoid, alkaloid, amino acid, fatty acid, quinone, essence oil of stem and leaf. As essence oil of full plant, methyl-n-nonyl-ketone was reported. In addition, there were reported that α-pinene, camphene, safrol, β-carophyllene, linalool and humulene(Choe, K. H., Yoon, C. H. and Kwon, S. J. (1988b) Chemical composition of Saururaceae growing in Korea - on volatile constituents of Saururus chinensis by GC/MS. Punsok kwahak 1: 259-262 and Choe, K. H., Kwon, S. J. and Lee K. C. (1989) Chemical composition of Saururaceae growing in Korea - on fatty acids and amino acids of Houttuynia cordata and Saururus chinensis. Punsok kwahak 2: 285-288). As flavonoid, the main ingredients of stem and leaf, hyperin, quercetin, isoquercitrin and quercitrin were reported. (Choe, K H., Yoon, C. H. and Kwon, S. J. (1994) A study of chemical constituents of Saururaceae growing in Korea - on flavonoid constituents of Saururus chinensis. Anal. Sci. Technol. 7: 11-15 Sung, S. H., Kwon, S. H., Cho, N. J. and Kim, Y. C. (1997) Hepatoprotective flavonolglycosides of Saururus chinensis Herbs. Phytotherapy Res. 11: 500-503 and Wang, E. C., Shih, M. H., Liu, M. C., Chen, M. T. and Lee, G. H. (1996) Studies on constituents of Saururus chinensis. Heterocycles 43: 969-972). Quinone series ingredients such as emodin, physcion and lignan such as carpanone were reported. (Crohare, R, Priestap, H. A., Farina, M., Cedola, M. and Ruveda, E. A. (1962) Aristolactams of Aristolochia argentina. Phytochemistry 13: 1957-1960). In addition, saurolactam, the aristolactam series alkaloid was isolated as cell toxic ingredient (Priestap, H. A. (1989) 13C-NMR spectroscopy of aristolochic acid and aristolactams. Magn. Reson. Chem. 27: 460-465).

Aristolactam, the same series alkaloid was also reported.(Crohare, R., Priestap, H. A., Farina, M., Cedola, M. and Ruveda, E. A. (1962) Aristolactams of Aristolochia argentina. Phytochemistry 13: 1957-1960). In addition, fatty acid such as cupric acid, linoleic acid and lauric acid, amino acid such as alanine, valine, glutamic acid, aspartic acid, leucine, isoleucine and proline were also reported. (Priestap, H. A. (1985a) Seven aristolactams from Aristolochia argentina. Phytochemstry 24: 849-852).

### Technical subject to be accomplished by the invention

The present inventors have studied extract of Saururus chinensis and materials isolated from the extract. Accordingly, the object of the present invention is to provide the use of a compound of the general formula (I) in the manufacture of medicines for prevention and treatment of stroke or Alzheimer's disease.

### Brief Description of the Drawing

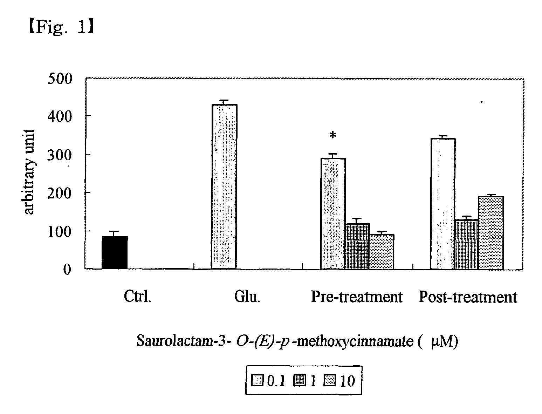

Fig. 1 is a graph showing the effect of saurolactam-3-O-(E)-p-methoxycinnamate on peroxide content on glutamate-insulted rat cortical cells in pre- or post-treatment.

### Constitution of the Invention

The constitution and effect of the present invention are explained in detail.

Extration of S. chinensis is carried out in lower alkanol such as methanol, ethanol or propanol, chlorinated hydrocarbon such as methylene chloride or chloroform, hydrocarbon such as hexane or heptane, and aromatic hydrocarbon such as benzene or toluene, or mixture thereof.

The present invention is detailedly explained by the following examples and experimental examples.

### Example 1

Dried S. chinensis (10kg) was extracted three times with CHCl₃-MeOH(1 : 1) in a reflux apparatus which, upon removal of the solvent in vacuo, yielded a total extract of S. chinensis(1.8kg. This extract was suspended in water and extracted with CH₂Cl₂. The CH₂Cl₂ extract was evaporated to dryness in vacuo. The resultant CH₂Cl₂ fraction was suspended in 90% MeOH and extracted with n-hexane. The n-hexane extract and the residual 90% MeOH suspension were dried in vacuo to yield 60g of n-hexane fraction and 120.0g of 90% MeOH fraction, respectively.

### Example 2

### Isolation and identification of compound 1

The n-hexane fraction(60g) was chromatographed on silicagel column eluted with a stepwise gradient from n-hexane fraction n-hexane -EtOAc(100 : 1) to give 20 subfractions. Subfraction 6 (600mg) was chromatographed on a Sephadex LH-20 column to give crude alkaloid fraction (200mg). Compound 1(Rt 14.15min) was isolated by reverse phase HPLC. Compound 1 was recrystallized with MeOH to give compound 1(100mg).

### Identification of compound 1

Yellowish powder
UV (CHCl₃) λ max (log ε): 388.10 (3.88), 314.20 (3.94), 287.20 (4.45), 276.50 (4.42), 262.10 (4.42), 235.50 (4.56) nm
IR (KBr) v max: 3220, 1701, 1648, 1427, 1319, 1257, 1031, 972, 835, 737, 604 cm⁻¹
EIMS (m/z) (rel. int.): 279 [M⁺] (100), 264 (31), 236 (25), 194 (61)
¹H NMR (300 MHz, DMSO-d6): δ 10.32 (OH), 9.10 (1 H, dd, J = 8.85, 2.50 Hz, H-5), 7.94 (1 H, dd, J = 8.16, 2.25 Hz, H-8), 7.62 (1 H, s, H-2), 7.58 (2 H, td, J = 7. 00, 7.25 Hz, H-6 and H-7), 7.28 (1 H, s, H-9), 4.01 (3 H, s, OCH₃), 3.38 (3 H, s, NCH₃) ppm
¹³C NMR (100 MHz, DMSO-d6): δ 166.79 (C-1), 152.17 (C-3), 148.81 (C-4), 136.93 (C-9a), 134.64 (C-4a), 129.01 (C-8), 127.44 (C-6), 126.86 (C-5), 126.30 (C-8a), 125. 57 (C-7), 120.96 (C-1a and 1b), 120.14 (C-5a), 113.60 (C-2), 103.48 (C-9), 59.52 (OCH₃), 26.17 (NCH₃) ppm

Based on the above chemical and spectrophotometrical results, compound 1 is identified as 10-aminomethyl-4-methoxy-3-hydroxy-phenanthrene-1-carboxylic acid lactam, saurolactam (Crohare, R., Priestap, H. A., Farina, M., Cedola, M. and Ruveda, E. A. (1962) Aristolactams of Aristolochia argentina. Phytochemistry 13: 1957-1960 Priestap, H. A. (1989) 13C-NMR spectroscopy of aristolochic acid and aristolactams. Magn. Reson. Chem. 27: 460-465 and Priestap, H. A. (1985a) Seven aristolactams from Aristolochia argentina. Phytochemstry 24: 849-852).

### Example 3

### Isolation and identification of Compound 2

During isolation of Compound 1, the peak of semi-prep. HPLC (Rt 15.90 min) is collected and compound 2(10mg) was obtained by the same way as compound 1.

### Identification of Compound 2

Yellowish powder
UV (CHCl₃) λ max (log ε): 380.00 (3.74), 314.20 (3.78), 285.20 (4.33), 275.00 (4.35), 261.20 (4.27), 230.00 (4.41) nm
IR (KBr) v max: 3220, 1701, 1648, 1427, 1319, 1257, 1031, 972, 835, 737, 604 cm⁻¹ EIMS (m/z) (rel. int): 279 [M⁺] (100), 264 (59), 236 (66)
¹H NMR (300 MHz, DMSO-d6): δ 10.85 (NH), 9.12 (1 H, dd, J = 8.32, 2.01 Hz, H-5), 7.94 (1 H, dd, J = 8.32, 2.01 Hz, H-8), 7.85 (1 H, d, J = 2.01 Hz, H-3), 7.57 (2 H, m, H-6 and H-7), 7.14 (1 H, s, H-9), 4.10 (3 H, s, OCH₃), 4.01 (3 H, s, OCH₃) ppm
¹³C NMR (100 MHz, DMSO-d6): δ 168.85 (C-1), 154.71 (C-3), 150.86 (C-4), 135.80 (C-9a), 135.30 (C-4a), 129.51 (C-8), 127.95 (C-6), 127.30 (C-5), 126.38 (C-8a), 125.96 (C-7), 123.78 (C-1a), 122.60 (C-1b), 120.75 (C-5a), 110.37 (C-2), 105.07 (C-9), 60.39 (OCH3), 57.40 (OCH3) ppm

Based on the above chemical and spectrophotometrical results, compound 2 is identified as 10-amino-3,4-methoxy-3-hydroxy-phenanthrene-1-carboxylic acid lactam, aristolactam BII (Crohare, R., Priestap, H. A., Farina, M., Cedola, M. and Ruveda, E. A. (1962) Aristolactams of Aristolochia argentina. Phytochemistry 13: 1957-1960 Priestap, H. A. (1989) ¹³C-NMR spectroscopy of aristolochic acid and aristolactams. Magn. Reson. Chem. 27: 460-465 and Priestap, H. A. (1985a) Seven aristolactams from Aristolochia argentina. Phytochemstry 24: 849-852).

### Example 4

### Synthesis and identification of Compound 3

Compound 3 was synthesized by esterification of saurolactam and E-p-methoxycinnamic acid. Saurolactam(10mg) and E-p-methoxycinnamic acid dissolved in anhydrous toluene and added dicyclohexylcarbodiimide and 4-dimethylaminopyridine. And these were stirred in oil bath at 70°C under Ar gas. After reaction for 18 hours, the solution was filtered for eliminating by-product, urea. The filtered solution was evaporated in vacuo and chromatographed on a silica gel column with step gradient from n-hexane : CH₂Cl₂ : MeOH to give compound 3.

### Identification of compound 3

Yellow powder
EIMS (m/z) (rel. int): 409 (17) [M⁺], 293 (16), 279 (14) [M⁺-cinnamic acid], 224 (9) 178 (100), 164 (72), 131(56)
¹H NMR (300 MHz, CDCl3): 9.10 (1 H, d, J = 8.32 Hz, H-5), 8.00 (1 H, d, J = 15.90 Hz, H-7'), 7.92 (1H, s, H-2), 7.87 (1 H, m, H-8), 7.69 - 7.55 (4 H, m, H-2', 3', 5', 6'), 7.45 (2 H, m, H-6 and H-7), 7.10 (1 H , s, H-9), 6.75(1H, d, J = 15.90 Hz, H-8'), 4.01 (3 H, s, OCH₃), 3.50 (3 H, s, NCH₃) ppm

Compound 3 was identified as saurolactam-3-O-E-p-methoxycinamate by physical and spectral data.
EIMS (m/z) (rel. int.): 409 (17) [M⁺], 293 (16), 279 (14) [M+-cinnamic acid], 224 (9) 178 (100), 164 (72), 131(56)
¹H NMR (300 MHz, CDCl₃): 9.10 (1 H, d, J = 8.32 Hz, H-5), 8.00 (1 H, d, J = 15.90 Hz, H-7'), 7.92 (1H, s, H-2), 7.87 (1 H, m, H-8), 7.69 - 7.55 (4 H, m, H-2', 3', 5', 6'), 7.45 (2 H, m, H-6 and H-7), 7.10 (1 H , s, H-9), 6.75(1H, d, J = 15.90 Hz, H-8'), 4.01 (3 H, s, OCH₃), 3.50 (3 H, s, NCH₃) ppm

### Experimental example

### Experimental method

1. Animal : The Sprague-Dawley rat obtained from the breeding ground of SeoulNational University were breeded at the breeding ground of College of Pharmacy, Seoul National University. The breeding ground was maintained 22±5°C. Lightening was performed at from 7 AM to 7 PM. Solid feed containing crude protein 23.2%, crude lipid 4.0%, crude fiber 6.0%, crude ash 10.0%, crude calcium 0.6% and crude P 0.4%(Seoul, Samyangsa) was used.

### 2. Cortical cell culture

Primary cultures of mixed cortical cells containing both neuron and glia were prepared from fetal rats as described previously (Kim YC, Kim SR, Markelonis GJ and Oh TH (1998) Ginsenosides Rb1 and Rg3 protect cultured rat cortical cells from glutamate-induced neurodegeneration. J. Neurosci. Res. 53: 426-432).
The cortical cells were plated onto collagen-coated dishes at a density of 1×10⁶ cells/ml. The cultured cells were grown in DMEM supplemented with 10% heat-inactivated fetal calf serum, 1mM sodium pyruvate, 100IU/ml penicillin and 100 µg/ml streptomycin at 37°C in a humidified atmosphere of 95% air/5% CO₂. Cultures were allowed to mature for 2 weeks before being used for experiments.

### 3. Administration of extract of S. chinensis

Total extract of S. chinensis, fraction and isolated materials were dissolved in DMSO(within final culture concentration, 0.1%) and diluted with distilled water and by passing the solution through Millipore membrane(0.22µm, Millex-GV, USA) to make sterile state. The solution was cultured by differentiating the concentration and dosed to neuron cells.

### 1) MTT measurements

To culture broth of the cortical cells during being cultured, MTT(5mg/ml) was added by 10% of the culture broth. The broth was cultured for 3 more hours. The formed formazan was dissolved by DMSO and was assayed absorption at 540nm. (Mosmann T (1983) Rapid colorimetric assay for cellular growth and survival: Application to proliferation and cytotoxicity assays. J. Immuno. Methods 65: 55-61).

### 2) Measurement of Nitric oxide

The content of nitrite isolated in culture broth from first cultured cortical cells was assayed by using Griess reagent by Dawson's method(Dawson VL, Brahbhatt HP, Mong JA and Dawson TM (1994) Expression of inducible nitric oxide synthase causes delayed neurotoxicity in primary neuronal-glial cortical cultures. Neuropharmacology 33: 1425-1430).

### 3) Measurement of Ca²⁺ concentration in cells

Ca²⁺ concentration of first cultured cortical cells was assayed by dosing samples to be tested and after one hour, neurotoxicity was induced by glutamate and tested by using Fura-2 AM(Grynkiewicz G, Poenie M and Tsien RY (1985) A new generation of calcium indicators with greatly improved fluorescence properties. J. Biol. Chem. 260: 3440-3450).

### 4) Results and Discussion

We employed primary cultures of rat cortical cells as an *in vitro* assay system to isolate neuroprotective compounds from natural products that protect against glutamate-induced neurotoxicity (Kim et al., 1998). In the course of screening of natural sources with protective effects against glutamate-induced injury in our culture, we have discovered that a CHCl₃ /MeOH extract from the roots of *Saururus chinensis* Ball (Saururaceae) exhibited significant neuroprotective activity (Table 1). Compound 1 and 2 were isolated and identified by column chromatography on Sephadex LH-20 and semi-preparative HPLC as saurolactam and aristolactam of the following formula.

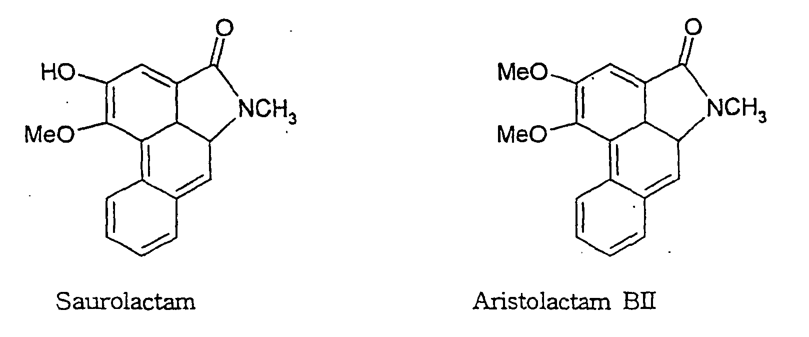

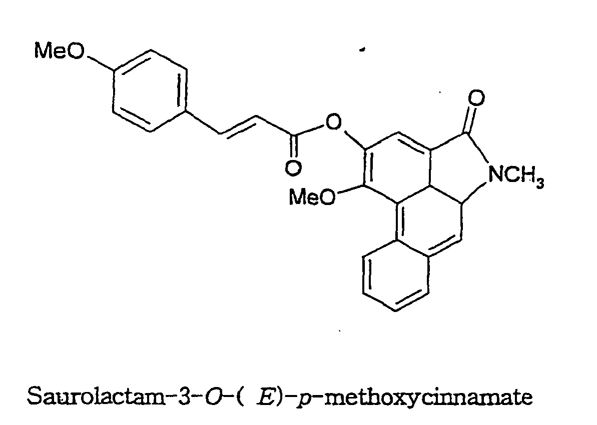

### Fig. 1. Structures of compounds 1 - 3

Bioassay-guided fractionation of a total extract of *S. chinensis* revealed that the *n*-hexane fraction was the most active one (50.6% protection against glutamate-induced toxicity at 10 g/ml, *p* < 0.001) (Table 1). Further fractionation and separation of the *n*-hexane fraction by several chromatographic methods yielded two compounds 1 and 2. Compounds 1 and 2 were identified as 10-aminomethyl-4-methoxy-3-hydroxy-phenanthrene-1-carboxylic acid lactam, saurolactam and 10-amino-3,4-methoxy-3-hydroxy-phenanthrene-1-carboxylic acid lactam, aristolactam BII by physical and spectral data comparison with those of published data (Crohare et al., 1962 Priestap et al., 1985a Priestap, 1989). The neuroprotective activity of saurolactam and aristolactam BII was further assessed in our culture system. After 24 hr incubation in DMEM in the presence of alkaloids, the cultures were assessed for the extent of neuronal damage (*treatment throughout) .* Saurolactam and anistolactam BII showed significant neuroprotective activity at the concentrations ranging from 1.0 to 10.0 M in *throughout treatment* paradigm (Table 2).

The neuroprotective activity of these two aristolactam alkaloids against glutamate-induced toxicity was initially evaluated by a timed exposure to alkaloids before or after excitotoxic challenge. Some cultures were pretreated with alkaloids for one hr before a brief exposure (30 min) to glutamate, washed and maintained in DMEM for 24 hr in the absence of alkaloids (*pre-treatment*) (Table 3). Also, cultures were first exposed to glutamate for 30 min, washed and maintained in DMEM for 24 hr in the presence of alkaloids .(*post-treatment*) (Table 4).

As shown in Table 4, these alkaloids significantly suppressed neurotoxicity induced by glutamate in *post-treatment* at concentrations ranging from 0.1 to 10 M. However, saurolactam and aristolactam BII did not significantly protect cultured cortical cells from glutamate-induced neurotoxicity in *pre-treatment* paradigm (Table 3). From these results, aristolactam alkaloids might not be directly antagonistic to glutamatergic receptor binding or to glutamatergic receptor-mediated cellular responses in early stage. These alkaloids were suggested they might act on the cellular responses in downstream after overactivation of glutamatergic receptor. This was further confirmed by our results showing that aristolactam alkaloids did not show any significant effect on increase of intracellular Ca²⁺ in early stage of glutamate-induced neurotoxicity (data not shown). An excessive intracellular neuronal Ca²⁺ concentration can activate a series of enzymes including nitric oxide synthase (NOS), protein kinase C, phospholipases and proteases. When NOS is activated, excess NO is generated. NO is known to be involved in glutamate neurotoxicity (Masanori *et al*., 1998). NO reacts with superoxide anion to form peroxynitrite radicals, which result in the production of dose-dependent neuronal damage (Almeida *et al*, 1998). Therefore, we estimated the effect of aristolactam BII, more potent neuroprotective alkaloid, on NO content in culture (Table 5). The cortical cultures treated with glutamate alone showed an increased formation of NO, whereas NO content in cultures treated with glutamate plus aristlactam BII was increased only slightly (Table 5). This result suggested that aristolactam BII may inhibit the overproduction of peroxynitrite radicals or the over-activation of NOS.

Glutamate receptor activation also stimulates phospholipase A2 (Lipton and Rosenberg, 1994). Activation of phospholipase A2 leads to generation of its metabolite, arachidonic acid. Arachidonic acid potentiates NMDA-evoked currents and inhibits reuptake of glutamate into astrocytes and neurons which further exacerbates the situation (Miller *et al*., 1992). Oxygen free radicals can be formed during arachidonic acid metabolism, leading to further phospholipase A2 activation (Lafon-Cazal *et al*., 1993). Another well-known mechanism of glutamate-induced neuronal degeneration is oxidative stress (Choi, 1988 Coyle and Puttfarcken, 1993). CNS neurons are unusually vulnerable to oxidative stress because of their high rate of oxygen consumption, and because of the high content of polyunsaturated fatty acids within cells of the nervous system (Buckmann *et al.,* 1993). Nevertheless, the brain has a cellular defense system which includes high levels of several antioxidant compounds (e.g. GSH) and several antioxidant enzymes (e.g. catalase, SOD, GSH-px and GSSG-R). Therefore, we determined the effect of aristolacam BII on the activities of the antioxidant enzymes. However, this alkaloid showed no significant protective effects on the activities of catalase, SOD and GSH-px (data not shown). Therefore, aristolactam BII could protect cultured cortical neurons from glutamate-induced neurotoxicity by directly acting on NOS. However, further experiments will be carried out to confirm this postulated mechanism.

On the other hand, we previously patented (*E*)-*p*-methoxycinnamic acid as a neuroprotectant agent 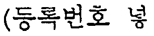 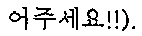 Therefore, we modified the chemical structure of aristolactam alkaloid in order to improve the neuroprotective activity of authentic aristolactam alkaloid. We synthesized saurolactam-3-*O*-(*E*)-*p*-methoxycinnamate (S-MCA) by chemically modifying saurolactam and *E*-*p*-MCA. The neuroprotective activity of S-MCA was evaluated using our *in vitro* culture system (Tables 6 and 7). S-MCA showed the significant neuroprotective activity on glutamate-induced neurotoxicity in both *pre-* and *post-treatment* paradigms. That is, S-MCA has the improved neuroprotective activity compared with aristolactam alkaloids showing significant protective activity only in *post-treatment* paradigm and *E*-*p*-MCA showing significant protective activity only in *pre-treatment* paradigm. Therefore, S-MCA is a highly capable candidate for the treatment and prevention of neurodegenerative disease.

This improved neuroprotective activity of S-MCA was further confirmed by our continuous experimental researches. S-MCA could successfully block the increase of intracellular Ca²⁺ induced by excess glutamate (Table 8). Furthermore, S-MCA could significantly block NO synthesis as a result of successful blocking the increase of intracellular Ca²⁺ in both *pre-* and *post-treatment* paradigms (Table 9). S-MCA also reduced cellular peroxide level in glutamate-injured cortical neurons

### 5) Inducement of neurotoxicity by glutamate

Cortical cells directly isolated from rat fetal were cultured for 14 days. The samples to be tested were treated at different concentrations to cultured cells. After one hour, neurotoxicity was induced by treating 100 µM of glutamate. After 24 hours, the survival cell rate was measured by MTT assay to determine neuroprotective activity of the samples.

### 6). Statistical analysis

The study for statistical significance was carried out with number 3 (n=3) for each test group. Data were evaluated for statistical significance using analysis variance (ANOVA test) The confidence level for statistical significance was set at a probablilty value of 5%.

**Table 1. Neuroprotective effect of each fraction of S. chinensis Herbs on cell viability in glutamate-insulted rat cortical cells in throughout treatment^{a}.**

| | Concentration | Viability |
|---|---|---|
| | (µg/ml) | (%) |
| Control | | 100.0 ± 4.2 |
| Glutamate-treated ^{b} | | 0.0 ± 1.3 |
| Total extract | 1 | 8.0 ± 0.5 |
| | 10 | 24.1 ± 1.2∗ |
| | 100 | 45.1 ± 1.5∗∗ |
| CH₂Cl₂ fr. | 1 | 1.0 ± 3.0 |
| | 10 | 27.6 ± 3.6∗ |
| | 100 | 52.6 ± 2.9∗∗ |
| n-Hexane fr. | 1 | 60.4 ± 1.8∗∗∗ |
| | 10 | 50.6 ± 0.7∗∗ |
| | 100 | 34.3 ± 2.0∗ |
| 90% MeOH fr. | 1 | 7.5 ± 0.8 |
| | 10 | 14.6 ± 1.7 |
| | 100 | 0.9 ± 2.2 |
| Aqueous fr. | 1 | no effect |
| | 10 | no effect |
| | 100 | no effect |
| n-BuOH fr. | 1 | no effect |
| | 10 | no effect |
| | 100 | no effect |

| | | |
|---|---|---|
| ^{a} Throughout treatment: combined treatment of pre-treatment and recovery only. ^{b} Glutamate-treated differs significantly from the control at a level of p< 0.001. | | |

Control is the value for primary cultures of rat cortical cells. Control value for MTT was 1.03±0.08 optical density (OD). Glutamate-treated is the value for primary cultures of rat cortical cells exposed to glutamate for 30 min. Glutamate-treated value for MTT was 0.58±0.02 OD.

Viability is calculated as 100 X (OD of sample treated - OD of Glutamate-treated)/(OD of control - OD of Glutamate-treated). Results differ significantly from the Glutamate-treated at a level of ∗: p<0.05, ∗∗: p<0.01, ∗∗:p<0.001.

**Table 2. Effects of saurolactam or aristolactam BII on cell viability in glutamate- insulted rat cortical cells in throughout treatment ^{a}.**

| | Concentration | Viability |
|---|---|---|
| | (µM) | (%) |
| Control | | 100.0 ± 4.2 |
| Glutamate-treated ^{b} | | 0.0 ± 1.3 |
| saurolactam | 0.1 | no effect |
| | 1.0 | 39.0 ± 1.8∗ |
| | 10.0 | 46.6 ± 2.1∗∗ |
| aristolactam BII | 0.1 | no effect |
| | 1.0 | 60.8 ± 2.1 ∗∗∗ |
| | 10.0 | 52.2 ± 3.2 ∗∗ |

| | | |
|---|---|---|
| ^{a} The experimental protocol, nomenclature and foot notes for Table 2 are precisely the same as described under the legend to Table 1. | | |

**Table 3.Neuroprotective effect of saurolactam or aristolactam BII on cell viability in glutamate-insulted rat cortical cells in pre-treatment ^{a}.**

| | Concentration | Viability |
|---|---|---|
| | (µM) | (%) |
| Control | | 100.0 ± 4.2 |
| Glutamate-treated^{b} | | 0.0 ± 1.3 |
| saurolactam | 0.1 | 0.0 ± 4.5 |
| | 1.0 | 0.0 ± 2.1 |
| | 10.0 | toxic effect |
| aristolactam BII | 0.1 | 0.0 ± 7.8 |
| | 1.0 | 0.0 ± 3.2 |
| | 10.0 | 32.2 ± 0.8∗ |

| | | |
|---|---|---|
| ^{a} Pretreatment: Sample treatment was conducted 1hr prior to the glutamate insult b Glutamate-treated differs significantly from the control at a level of p < 0.001. | | |

The nomenclature and foot notes for Table 3 are precisely the same as described under the legend to Table 1.

**Table 4.Neuroprotective effects of saurolactam or aristolactam BII on cell viability on glutamate-insulted rat cortical cells in post-treatment ^{a}.**

| | Concentration | Viability |
|---|---|---|
| | (µM) | (%) |
| Control | | 100.0 ± 4.2 |
| Glutamate-treated ^{b} | | 0.0 ± 1.3 |
| saurolactam | 0.1 | 0.0 ± 2.9 |
| | 1.0 | 66.3 ± 2.1 ∗∗ |
| | 10.0 | 32.1 ± 1.9 ∗ |
| aristolactam BII | 0.1 | 58.5 ± 5.8 ∗∗ |
| | 1.0 | 68.4 ± 3.5 ∗∗∗ |
| | 10.0 | 47.8 ± 2.9 ∗∗ |

| | | |
|---|---|---|
| ^{a} Post-treatment: Sample treatment was conducted right after the 30 min-glutamate insult. ^{b} Glutamate-treated differs significantly from the control at a level of p < 0.001. | | |

The nomenclature and foot notes for Table 4 are precisely the same as described under the legend to Table 1.

**Table5. The effect of aristolactam BII on NO content released from glutamate- insulted rat cortical cells in post-treatment^{a}.**

| | Concentration | Nitrite |
|---|---|---|
| | (µM) | (nM) |
| Control | | 75.5±2.7 |
| Glutamate-treated ^{b} | | 145.7±5.2 |
| aristolactam BII | 0.1 | 87.0±0.8∗∗ |
| | 1.0 | 86.8±2.2∗∗ |
| | 10.0 | 103.2±1.2∗ |
| N^{ω}-nitro-L-arginine^{c} | 1.0 | 103.9±3.5∗ |
| | 10.0 | 88.5±4.1∗∗ |
| | 100.0 | 109.7±1.9∗ |

| | | |
|---|---|---|
| ^{a} The experimental protocol, nomenclature and foot notes for Table 5 are precisely the same as described under the legend to Table 4. ^{b} Glutamate-treated differssignificantly from the control at a level of p < 0.01. c N-nitro-L-arginine: nitric oxide synthase inhibitor | | |

**Table6.Neuroprotective effect of saurolactam-3-O-(E)-p-methoxycinnamate on cell viability in glutamate-insulted rat cortical cells in pre-treatment^{a}.**

| | Concentration | Viability |
|---|---|---|
| | (µM) | (%) |
| Control | | 100.0 ± 4.2 |
| Glutamate-treated ^{b} | | 0.0 ± 1.3 |
| saurolactam-3-O-(E)- | 0.1 | 29.8 ± 8.2 ∗ |
| p-methoxycinnamate | 1.0 | 67.2 ± 9.6 ∗∗∗ |
| | 10.0 | 80.6 ± 9.5 ∗∗∗ |

| | | |
|---|---|---|
| ^{a} Pre-treatment: Sample treatment was conducted 1hr prior to the glutamate insult. ^{b} Glutamate-treated differs significantly from the control at a level of p < 0.001. | | |

The nomenclature and foot notes for Table 6 are precisely the same as described under the legend to Table 1.

**Table7.Neuroprotective effect of saurolactam-3-O-(E)-p-methoxycinnamate on cell viability on glutamate-insulted rat cortical cells in post-treatment^{a}.**

| | Concentration | Viability |
|---|---|---|
| | (µM) | (%) |
| Control | | 100.0 ± 4.2 |
| Glutamate-treated ^{b} | | 0.0 ± 1.3 |
| saurolactam-3-O- | 0.1 | 62.1 ± 1.8∗∗∗ |
| (E)-p-methoxycinnamate | 1.0 | 70.3 ± 3.2∗∗∗ |
| | 10.0 | 48.2 ± 1.5∗∗ |

| | | |
|---|---|---|
| ^{a} Post-treatment: Sample treatment was conducted right after the 30 min-glutamate insult. ^{b} Glutamate-treated differs significantly from the control at a level of p < 0.001. | | |

The nomenclature and foot notes for Table 7 are precisely the same as described under the legend to Table 1.

**Table 8. The effect of saurolactam-3-O-(E)-p-methoxycinnamate on [Ca²⁺]i on glutamate- insulted rat cortical cells in pre-treatment^{a}.**

| | Concentration | [Ca²⁺]i |
|---|---|---|
| | (µM) | (nM) |
| Control | | 75.5 ± 15.0 |
| Glutamate-treated ^{b} | | 425.4 ± 20.0 |
| saurolactam-3-O- | 0.1 | 233.3 ± 14.9 ∗∗∗ |
| (E)-p-methoxycinnamate | 1.0 | 145.8 ± 26.3 ∗∗∗ |
| | 10.0 | 96.1 ± 4.1 ∗∗∗ |

| | | |
|---|---|---|
| ^{a} Pre-treatment : Sample treatment was conducted 1hr prior to the glutamate insult. ^{b} Glutamate-treated differs significantly from the control at a level of p < 0.001. | | |

The nomenclature and foot notes for Table 8 are precisely the same as described under the legend to Table 1.

**Table9. The effect of saurolactam-3-O-(E)-p-methoxycinnamate on NO content on glutamate-insulted rat cortical cells in pre^{a} - or post-treatment^{b}.**

| | Concentration | Nitrite (nM) | |
|---|---|---|---|
| | (µM) | pre-treatment | post-treatment |
| Control | | 70.5 ± 15.0 | |
| Glutamate-treated ^{c} | | 165.9 ± 10.5 | |
| saurolactam-3-O- (E)- | 0.1 | 125.0 ± 6.8∗ | 143.6 ± 5.7 |
| p-methoxycinnamate | 1.0 | 74.8 ± 6.5∗∗ | 97.5 ± 26.3∗∗ |
| | 10.0 | 71.4 ± 2.1∗∗ | 118.1 ± 2.1∗∗ |

| | | | |
|---|---|---|---|
| ^{a} Pre-treatment : Sample treatment was conducted 1hr prior to the glutamate insult. ^{b} Post-treatment: Sample treatment was conducted right after the 30 min-glutamate insult ^{c} Glutamate-treated differs significantly from the control at a level of p < 0.01. | | | |

The nomenclature and foot notes for Table 8 are precisely the same as described under the legend to Table 1.

The present compound of general formula (I) can be administered 1mg-200mg/day, 1-3 times. That can be varied by weight, sex, age and severity of disease of a patient.

The compound of the general formula (I) of the present invention can be used for prevention and treatment of neurodegenerative disorders. The compound of the present invention can be formulated with conventional vehicle into pharmaceutical preparation such as injection, solution, syrup, tablet or capsule by a conventional method.

The present invention is more detailedly explained by the following preparation examples.

### Preparation example 1

| | |
|---|---|
| Extract of S. chinensis | 100mg |
| distilled water for injection | QS |
| pH adjuster | QS |

The extract of S. chinensis is dissolved in some distilled water for injection and the mixture is adjusted with pH adjuster to about pH 7.6. Distilled water for injection is poured to the mixture to make 2ml and was filled in a 2ml ampoule.

### Preparation example 2

| | |
|---|---|
| compound 1 | 2mg |
| distilled water for injection | QS |
| pH adjuster | QS |

The compound 1 is dissolved in some distilled water for injection and the mixture is adjusted with pH adjuster to about pH 7.6. Distilled water for injection is poured to the mixture to make 2ml and was filled in a 2ml ampoule.

### Preparation example 3

| | |
|---|---|
| Extract of S. chinensis | 10mg |
| lactose | 100mg |
| starch | 100mg |
| magnesium stearate | QS |

The above ingredients are mixed and made into a tablet by a conventional tablet method.

### Preparation example 4

| | |
|---|---|
| compound 2 | 10mg |
| lactose | 100mg |
| starch | 50mg |
| magnesium stearate | QS |

The above ingredients are mixed and made into a tablet by a conventional tablet method.

### Preparation example 5

| | |
|---|---|
| Extract of S. chinensis | 5mg |
| lactose | 50mg |
| starch | 50mg |
| talc | 2mg |
| magnesium stearate | QS |

The above ingredients are mixed and filled in a gelatine capsule by a conventional capsule method.

### Preparation example 6

| | |
|---|---|
| compound 1 | 5mg |
| lactose | 100mg |
| starch | 93mg |
| talc | 2mg |
| magnesium stearate | QS |

The above ingredients are mixed and filled in gelatine capsule by a conventional capsule method.

### Preparation example 7

| | |
|---|---|
| Extract of S. chinensis | 50mg |
| suger | 20g |
| isomerized sugar | 20g |
| lemon essence | QS |
| distilled water | to make 100ml |

The above ingredients are mixed and filled in 100ml bottle and sterilized by a conventional solution method.

### Preparation example 8

| | |
|---|---|
| compound 3 | 50mg |
| suger | 20g |
| isomerized sugar | 20g |
| lemon essence | QS |
| distilled water | to make 100ml |

The above ingredients are mixed and filled in 100ml bottle and sterilized by a conventional solution method.

### Industrial applicability

From the above experiments total extract of S. chinensis, fraction of chlorinated hydrocarbon, n-hexane fraction and aristolactam series alkaloid and cinnamic acid derivatives can be used for the prevention and treatment of neurodegenerative disorder, such as stroke or Alzheimer's disease.

## Claims

1. Use of a compound of the general formula (I) in the manufacture of medicines for prevention and treatment of stroke or Alzheimer's disease 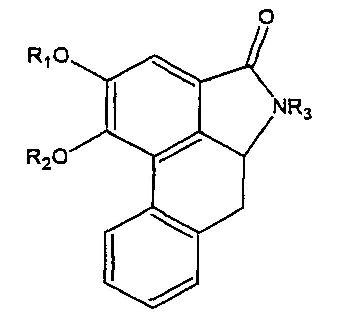 wherein R₁ is H, C₁₋₄ alkyl or the group of 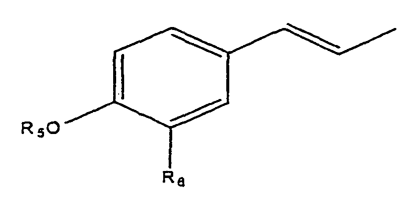 (in which R₅ is H or C₁₋₄ alkyl, R₆ is H, OH or C₁₋₄ alkyl),
R₂ is C₁₋₄ alkyl and
R₃ is C₁₋₄ alkyl.

## Patentansprüche

1. Verwendung einer Verbindung der allgemeinen Formel (I) zur Herstellung von Arzneimitteln zur Vorbeugung und Behandlung von Schlaganfall oder Alzheimer-Krankheit 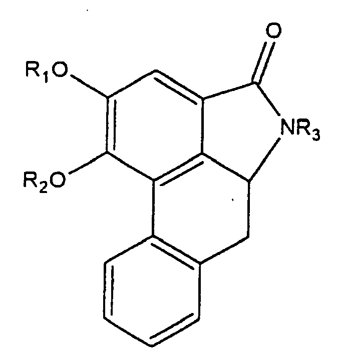 wobei R₁ H, C₁₋₄-Alkyl oder die Gruppe 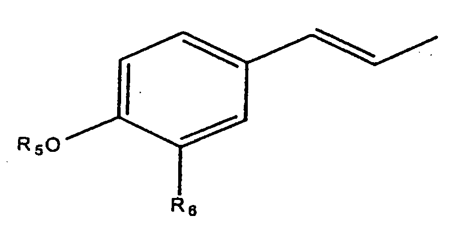 ist (in der R₅ H oder C₁₋₄-Alkyl ist, R₆ H, OH oder C₁₋₄-Alkyl ist),
R₂ C₁₋₄-Alkyl ist und
R₃ C₁₋₄-Alkyl ist.

## Revendications

1. Utilisation d'un composé de formule générale (I) dans la fabrication de médicaments pour la prévention et le traitement de l'accident cérébrovasculaire ou de la maladie d'Alzheimer, 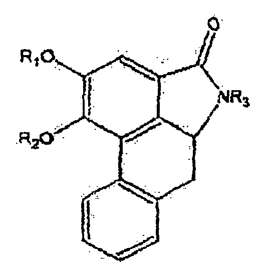 dans laquelle
- R₁ représente H, un groupe alkyle C₁₋₄ ou le groupe 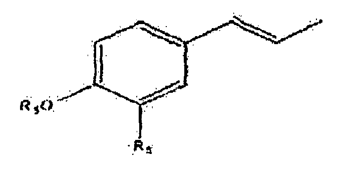 (dans lequel R₅ représente H, ou un groupe alkyle C₁₋₄, R₆ représente H, OH ou un groupe alkyle C₁₋₄),
- R₂ représente un groupe alkyle C₁₋₄, et
- R₃ représente un groupe alkyle C₁₋₄.
